# EUROPÄISCHE PATENTSCHRIFT

(11) **EP 0 377 127 B1**
(45) Veröffentlichungstag und Bekanntmachung des Hinweises auf die Patenterteilung: **08.03.1995**
(21) Anmeldenummer: 89122492.5
(22) Anmeldetag: 06.12.1989
(51) Int. Cl.: A61K 31/70

(54) **Verwendung von Myricetin-3-0-beta-D-glucuronid als Arzneimittel**
Use of Myricetin-3-O-beta-D glucuronide as medicament
Utilisation du Myricétine-3-O-bêta-D-glucuronide comme médicament

(30) Priorität: 02.01.1989 DE 3900023
(43) Veröffentlichungstag der Anmeldung: 11.07.1990
(73) Patentinhaber: Plantamed Arzneimittel GmbH, 92318 Neumarkt (DE)
(72) Erfinder: Hiermann, Alois, Dr., AT-8020 Graz (AT)
(74) Vertreter: Matschkur, Götz, Lindner Patent- und Rechtsanwälte

(56) Entgegenhaltungen:
- EP-A- 0 234 019
- CHEMICAL ABSTRACTS, Band 110, Nr. 1, 2. Januar 1989, Seite 435, Zusammenfassung Nr. 4676n, Columbus, Ohio, US; G. KITANOV: "A biflavone and flavanol and xanthone glycosides from Hypericum aucheri", & KHIM. PRIR. SOEDIN. 1988, (3), 454-5
- CHEMICAL ABSTRACTS, Band 106, Nr. 9, 2. März 1987, Seite 345, Zusammenfassung Nr. 64328c, Columbus, Ohio, US; V. CHEYNIER et al.: "HPLC separation and characterization of flavonols in the skins of Vitis vinifera var. Cinsault", & AM. J. ENOL. VITIC. 1986, 37(4), 248-52
- CHEMICAL ABSTRACTS, Band 100, Nr. 25, 18. Juni 1984, Seite 323, Zusammenfassung Nr. 206545y, Columbus, Ohio, US; J.B. LOWRY et al.: "Flavonol glycoside distribution in cultivars and hybrids of Leucaena leucocephala", & J. SCI. FOOD AGRIC. 1984, 35(4), 401-7
- CHEMICAL ABSTRACTS, Band 94, Nr. 21, 25. Mai 1981, Seite 386, Zusammenfassung Nr. 171008d, Columbus, Ohio, US; M.F. ABD-ALLA et al.: "Flavonoid glycosides and the chemosystematics of Eucalyptus camaldulensis", & PHYTOCHEMISTRY 1980, 19(12), 2629-32

## Beschreibung

Die Erfindung geht aus von der bereits aus EP A 234019 bekannten Eignung von wässrigen Auszügen von Epilobium angustifolium L. zur Hemmung der Metabolisierung der Arachidon-Säure, insbesondere zur Hemmung der Prostaglandin-Synthese und/oder -freisetzung bei der Behandlung von Krankheiten, die - ganz oder teilweise - auf der Freisetzung von Metaboliten der Arachidon-Säure beruhen oder von dieser beeinflußt werden. Voraussetzung hierfür war die intensive Bearbeitung der Pharmakologie und Phytochemie von Epilobium, einer Offizinalpflanze, deren Verwendung zunächst vornehmlich als Tee bekannt geworden ist. Auf Epilobium stieß man bei der Suche nach Substanzen, die eine akute Entzündung und Prostaglandin-Synthese und die Freisetzung der Prostaglandine hemmen, ohne jedoch die vielfach gravierenden Nebenwirkungen anderer auf die Verminderung der Entzündungsreaktionen und deren Folgeerscheinung hinwirkenden Stoffe aufzuweisen. Bei der primären Metabolisierung der Arachidon-Säure durch die Cyclooxygenase gelangt man u. a. zu den verschiedenen Prostaglandinen. Diese spielen eine wesentliche Rolle bei Entstehung und Ablauf einer akuten Entzündung. Sie steigern die Kapilarpermeabilität, sensibilisieren die Schmerzrezeptoren und setzen lysosomale Enzyme frei. Bei jeder Irritierung des Gewebes erfährt die normalerweise auf einem niedrigen Niveau liegende Prostaglandin-Synthese eine starke Steigerung. Auch im zentralen Nervensystem spielen die Prostaglandine eine wichtige Rolle, wie z. B. bei der Fieberentstehung. Zusätzlich hemmen sie die Spontanlipolyse und die Lipolyse, die durch Katecholamine, Glucagon oder Theophyllin stimuliert wird. Den Prostaglandinen kommt schließlich eine bedeutende Funktion bei der Steuerung des Gefäß-Blut-Organ-Systems zu.

Die Erfindung verfolgt nicht nur das Ziel, einen Stoff zu finden, der mit der Hemmung der Prostaglandin-Synthese und -freisetzung eine positive Einwirkung auf Entzündungszustände ausübt, sondern sie beabsichtigt die Bereitstellung eines chemischen Stoffes, der bisher noch nicht beschrieben und in seiner Wirksamkeit untersucht worden ist. Damit beschränkt sich die Erfindung nicht allein darauf, die Verwendung eines Stoffes zu dem oben erläuterten speziellen oder einem anderen pharmazeutischen Zweck aufzuzeigen. Diese Aufgabe wird gemäß der Erfindung gelöst durch ein Myricetin-3-0-β-D-glucuronid der allgemeinen Formel 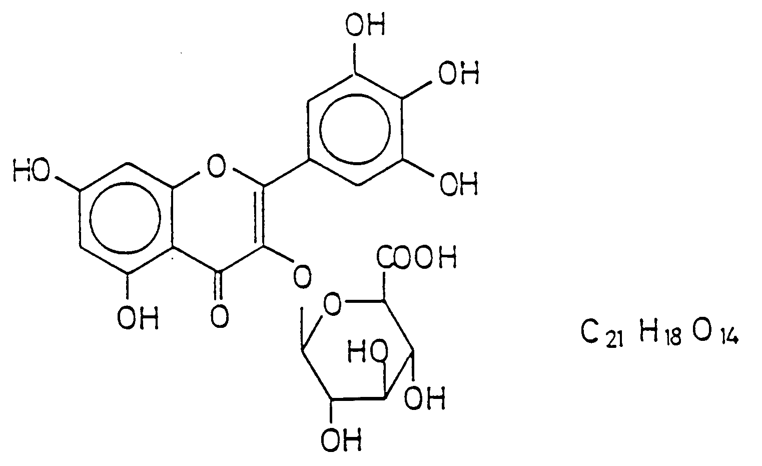
im folgenden als HIMAK bezeichnet.

HIMAK ist eine bekannte Verbindung. Sie ist in CA 1̅1̅0̅:4676n, CA 1̅0̅6̅:64328c, CA 1̅0̅0̅:206545y und CA 9̅4̅:171008d beschrieben.

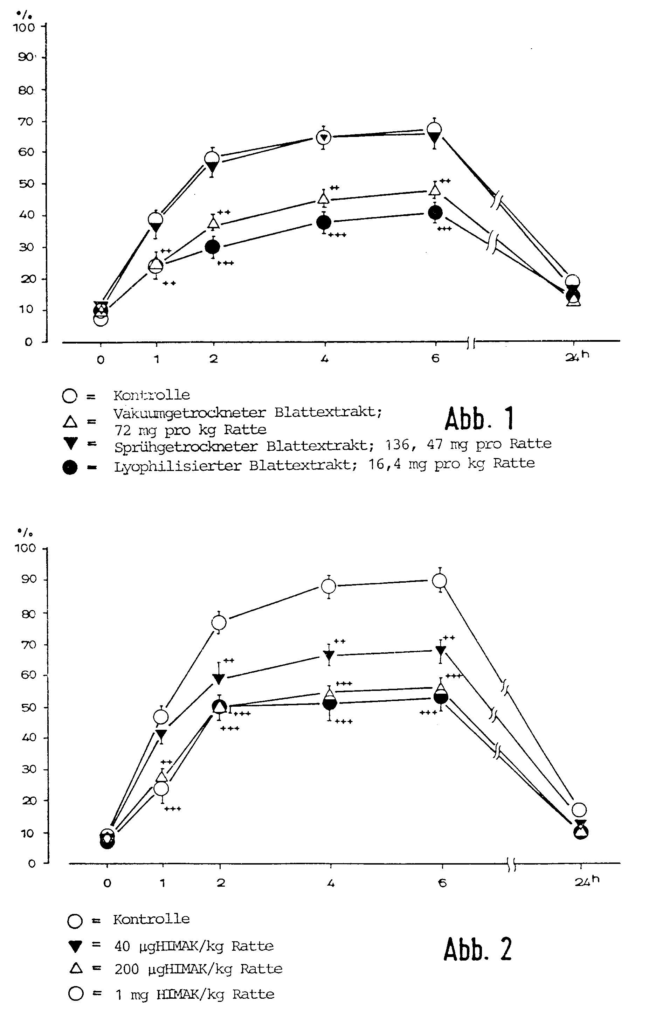

Zur Prüfung der entzündungshemmenden Wirkung dieser Substanz wird das Modell des Carrageenin-induzierten Rattenpfotenödems herangezogen, ein allgemein anerkanntes Modell zur Ermittlung anhand einer akuten Entzündung. Die entzündungshemmende Wirkung von vakuumgetrockneten und lyophilisierten Extrakten ist in Abb. 1 wiedergegeben. Bei der Anwendung von vakuumgetrocknetem Blattextrakt von Epilobium angustifolium wurde eine Dosis von 72 mg/kg Ratte, für sprühgetrockneten Blattextrakt eine Dosis von 136,47 mg/kg Ratte und für lyophilisierten Blattextrakt eine Dosis von 16,4 mg/kg Ratte eingesetzt. Das Ausmaß der entzündungswirkenden von vakuumgetrockneten und lyophilisierten Extrakten der in Abbildung 1 aufgezeigten Konzentrationen entspricht ca. 1 bis 2 mg Indometacin/kg Ratte.

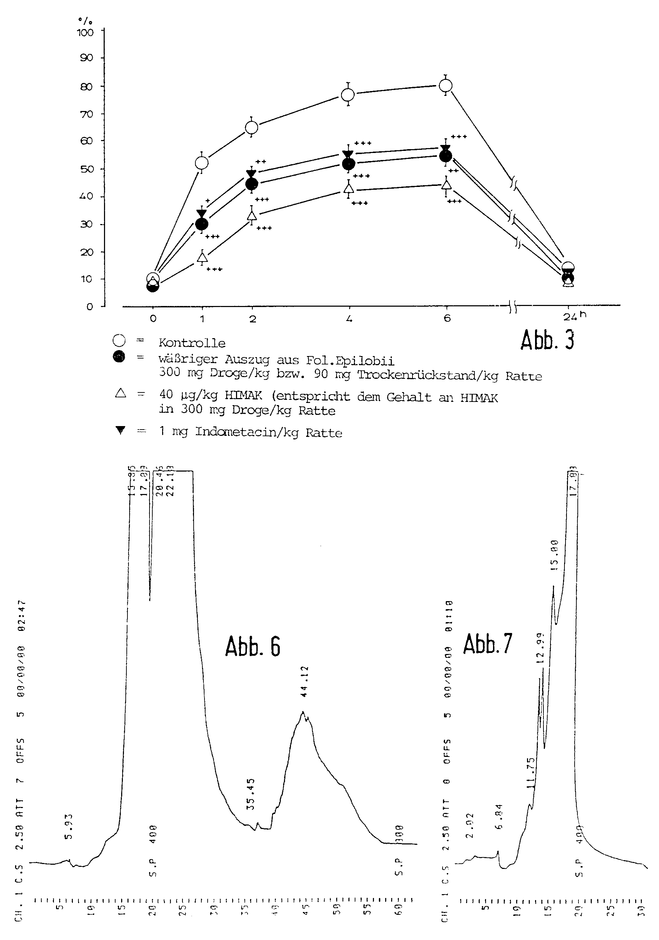

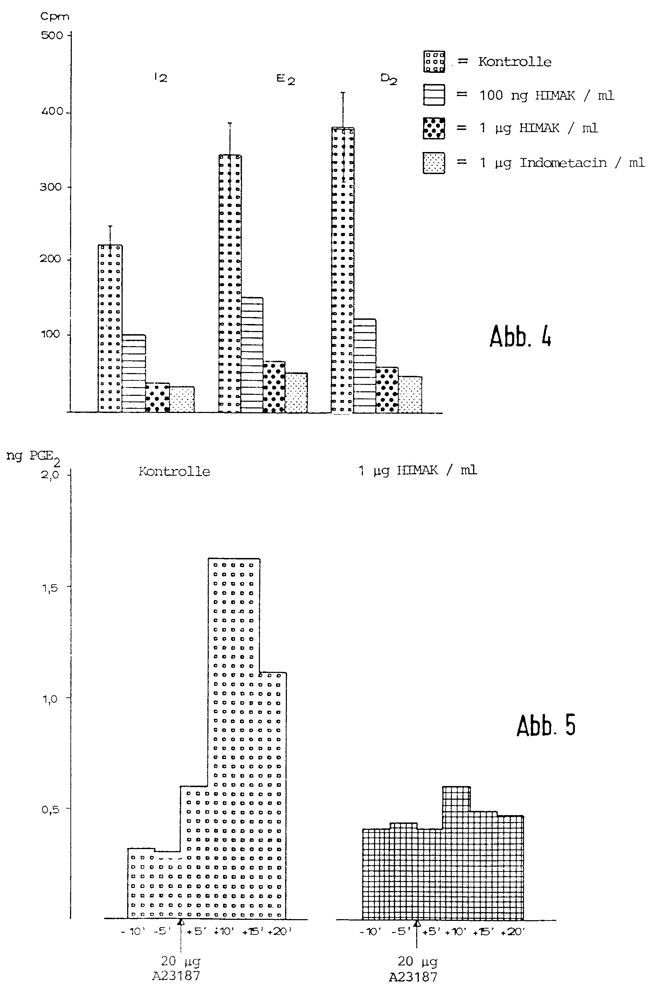

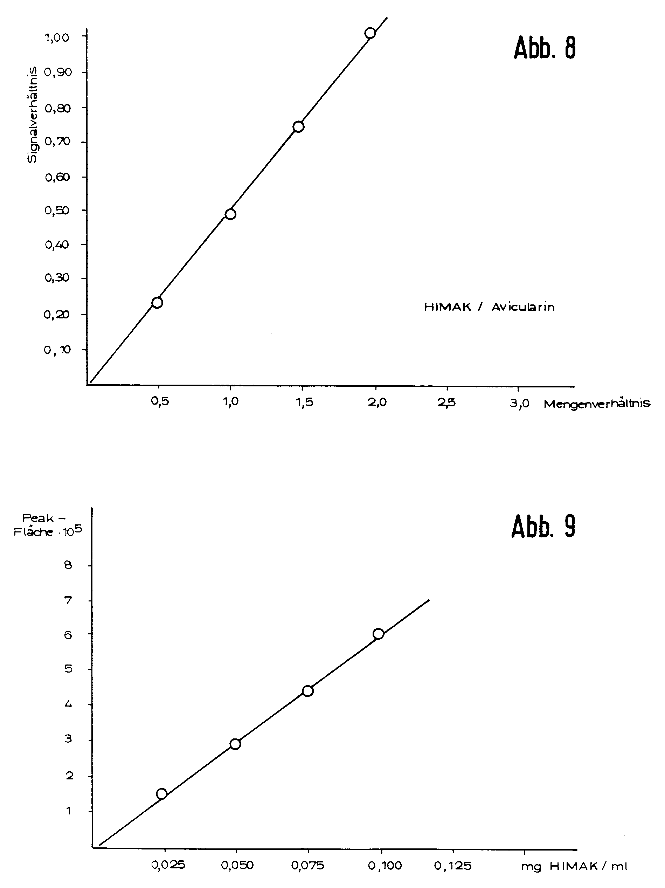

Der Einfluß auf das Carrageenin-induzierte Rattenpfotenödem geht aus den Kurven in den Abbildungen 2 und 3 hervor. Abbildung 2 stellt unterschiedliche Dosen von HIMAK einander gegenüber, Abb. 3 dient dem Vergleich von HIMAK einer bestimmten Dosis mit einem wässrigen Auszug von Epilobium und Indometacin. Der Darstellung des Einflusses der erfindungsgemäßen Substanz auf die Prostaglandin-Biosynthese am pharmakologischen Modell nach ISAKSON, modifiziert nach JUAN und SAMETZ am perfundierten Kaninchenohr sowie mittels Radioimmunoassay (RIA) dienen die Abbildungen 4 und 5.

Die angestellten Versuche zeigen ohne Ausnahme, daß wässrige Extrakte von Epilobium angustifolium, gereinigte Extrakte, Lyophilisate sowie die nahezu reine Wirksubstanz HIMAK dosisabhändig die Biosynthese von Prostaglandinen und eine experimentelle akute Entzündung (Rattenpfotenödem mit Carrageenin) deutlich hemmen.

Vergleiche mit Indometacin (Indo) ergeben folgendes Bild:
1. Die PG-Biosynthese wird durch HIMAK auf Gewichtsbasis in ähnlicher Konzentration wie durch Indo in etwa gleichem Ausmaß gehemmt (s. Abb. 4).
   1 »g/ml (Endkonzentration) Indo und ca. 1 »g/ml HIMAK hemmen die Biosynthese von PGI₂ und PGE₂ zu mehr als 80 % (siehe Abb. 5).
   Methode: Perfundiertes Kaninchenohr mit C-Arachidonsäure, Vormarkierung und Messung der radioaktiven Metalboliten mittels Auftrennung durch DC.
2. Entzündungshemmung
   Das Lyophilisat zeigt die erwartete, doch im Hinblick auf die Dosis überraschend hohe Wirksamkeit. 16.5 mg/kg des Lyophilisats sind ähnlich wirksam wie etwa 1 - 2 mg/kg Indo.

Die Reinsubstanz HIMAK zeigt sich im Vergleich mit der Referenzsubstanz Indo in den ersten Versuchen auf Gewichtsbasis als wesentlich wirksamer.

1 mg/kg Indomethacin (p.o.) reduziert die Entwicklung des Carrageenin-Pfotenödems ähnlich stark wie HIMAK im Dosisbereich von 40 - 200 »g/kg KG. Das heißt, die Reinsubstanz scheint auf Gewichtsbasis zumindest etwa 10 mal, auf geschätzter Molekulargewichtsbasis etwa 5 mal so wirksam zu sein wie Indo.

Zwischen der Wirkung auf die PG-Biosynthese einerseits und und der Wirkung auf die akute experimentelle Entzündung andererseits gibt es einen klaren Unterschied im Vergleich mit Indo: Etwa gleich große Konzentration von Indo und HIMAK hemmen die PG-Biosynthese nahezu identisch. Die Entzündung wird jedoch durch HIMAK in wesentlich kleineren Dosen als durch Indo gehemmt. Daraus folgt, daß die entzündungshemmende Wirkung von HIMAK nicht allein auf die PG-Biosynthese-Hemmung zurückzuführungen ist, vielmehr noch ein weiterer Wirkungsmechanismus hinzukommt.

Es ist gesicherte Erkenntnis, daß Cyclooxygenase-Hemmer, also Hemmer der Biosynthese von Prostaglandinen, wie etwa Indo, die Magenschleimhaut schädigen können. Vorläufige Untersuchungen haben ergeben, daß 1 mg/kg Indo zur Bildung kleiner Erosionen in der Magenschleimhaut führt. Bei einer ebenfalls peroralen Gabe von 1 mg/kg HIMAK ist dies nicht der Fall. Im Ergebnis liefern diese Untersuchungen einen Hinweis auf die wesentlich günstiger zu beurteilende Frage der Toxizität von HIMAK.

Die Erfindung umfaßt darüber hinaus die Einstellung eines Extraktes von Epilobium angustifolium L. auf den Wirkstoff nach der oben angegebenen allgemeinen Formel sowie schließlich die Anwendung des Wirkstoffs bzw. des diesen enthaltenden Extraktes in den üblichen möglichen Arzneiformen.

## Patentansprüche

1. Myricetin-3-0-β-D-glucoronid der allgemeinen Formel 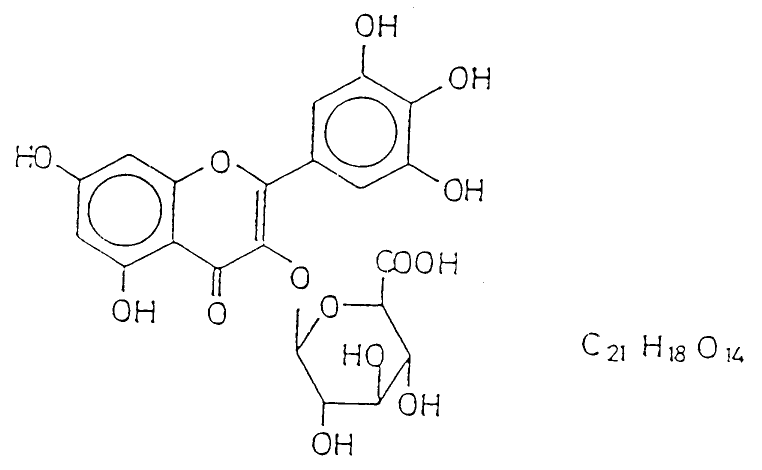 zur Verwendung als Wirkstoff zur Hemmung der Prostaglandin-Synthese und/oder -freisetzung bei der Behandlung von Krankheiten, insbesondere Entzündungen.

## Claims

1. Myricetin-3-0-β-D-glucoronid of the general formula 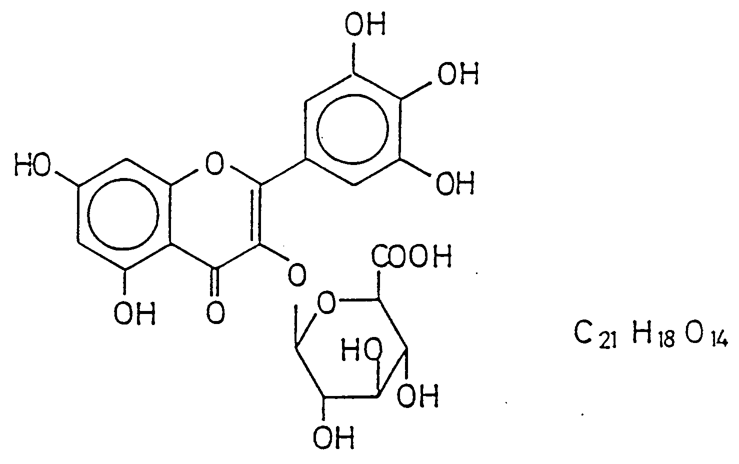 for the use as active ingredient for the inhibition of the synthesis and/or the releasing of Prostaglandin in course of the treatment of illnesses, especially of inflammations.

## Revendications

1. Myricetin-3-0-β-D-glucoronid de la formule générale 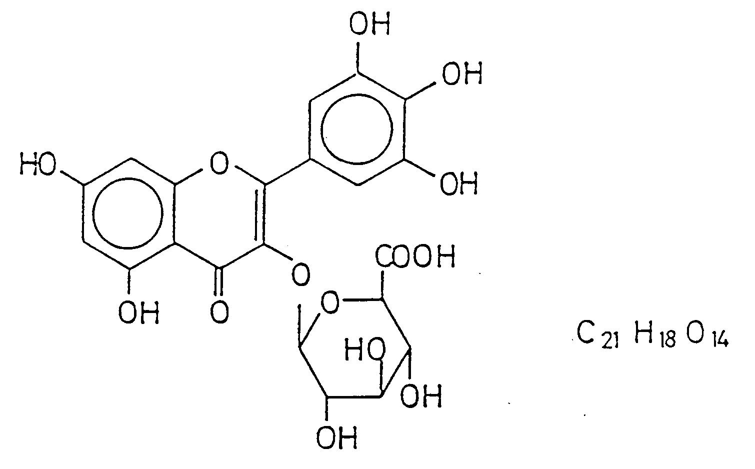 pour l'usage comme agent pour l'inhibition de la synthèse et/ou le dégagement de Prostaglandin pour le traitement des maladies, especialement des inflammations.
